(19) Europäisches Patentamt
European Patent Office
Office européen des brevets

(11) Veröffentlichungsnummer: **0 259 598**
**A2**

(12) **EUROPÄISCHE PATENTANMELDUNG**

(21) Anmeldenummer: 87111149.8

(51) Int. Cl.⁴: **C12P 19/26**

(22) Anmeldetag: 01.08.87

(30) Priorität: 08.08.86 DE 3626915

(43) Veröffentlichungstag der Anmeldung: 16.03.88 Patentblatt 88/11

(84) Benannte Vertragsstaaten: AT BE CH DE ES FR GB GR IT LI LU NL SE

(71) Anmelder: **HOECHST AKTIENGESELLSCHAFT**
Postfach 80 03 20
D-6230 Frankfurt am Main 80(DE)

(72) Erfinder: Thiem, Joachim, Prof. Dr.
Asbeckweg 31
D-4400 Münster(DE)
Erfinder: Treder, Wolfgang
Grosse Helkamp 16
D-4400 Münster(DE)
Erfinder: Keller, Reinhold, Dr.
Wiesenweg 5
D-6232 Bad Soden am Taunus(DE)
Erfinder: Schlingmann, Merten, Dr.
Schneidhainer Strasse 32a
D-6240 Königstein/Taunus(DE)

# (54) Verfahren zur enzymatischen Synthese eines N-Acetyl-neuraminsäure-haltigen Trisaccharids.

(57) Das Trisaccharid Neu-5-Ac-α(2→6)Gal-β(1→4)GlcNAc kann nach Bereitstellung der Ausgansprodukte Cytidin-5'-monophosphosialat und N-Acetyllactosamin enzymatisch mit Hilfe von Sialyltransferase in guten Ausbeuten hergestellt werden.

EP 0 259 598 A2

## Verfahren zur enzymatischen Synthese eines N-Acetylneuraminsäure-haltigen Trisaccarids

Das N-Acetylneuraminsäure-haltige Trisaccharid [Neu 5 Ac α(2→6) Gal β(1→4)GlcNAC] kommt als terminale Struktur des Haptens im Glycoprotein Glycophorin A auf der Oberfläche von Erythrozyten vor. Es kann als Immunalsorbens zur Isolierung von Antikörpern oder Krankheitserregern Anwendung finden, wenn es an eine Matrix gebunden ist.

Die chemische Synthese einer mit Schutzgruppen versehenen Vorläuferstufe des Trisaccharids gelang Paulsen 1984 [H. Paulsen u. H. Tietz, Carbohydr. Res. 125, 47 (1984)] $^1$H-NMR-spektroskopische Daten finden sich bei Vliegenthart et al., der an Asparaginsäure gebundene Oligosaccharide mit diesem Trisaccharid-Fragment aus Naturstoffen untersucht hat. [J.F.G. Vliegenthart, Adv. Carbohydr. Chem. 41, 209 (1983)] Die generelle Problematik bei der chemischen Synthese solch komplizierter Moleküle liegt in der hohen Stufenzahl (hier ca. 20), die durch Einführung von Schutzgruppen sowie Deblockierungsreaktionen zustande kommt, und in der geringen Stereospezifität solcher Synthesen, die sich in der Weise manifestiert, daß man bei Glycosylierungsreaktionen meist α, β-Anomerengemische erhält. Über die Biosynthesewege der Glycoproteine liegen umfangreiche Darstellungen vor [M.I. Horowitz et al. (Eds.): The Glycoconjugates, Vol. I - IV, Academic Press, New York 1977-1982].

Es wurde nun gefunden, daß das Trisaccharid nach Bereitstellung der Ausgangsprodukte enzymatisch in hoher Ausbeute nach dem in der Abbildung dargestellten Weg hergestellt werden kann.

Dies ist insofern überraschend, als es bisher noch nicht gelungen ist, das Trisaccharid auf enzymatischem Weg außerhalb der Zelle in guten Ausbeuten zu produzieren. Es konnte auch nicht erwartet werden, daß das erfindungsgemäße Verfahren so erfolgreich ist, da, wenngleich der Biosyntheseweg zum Vorbild genommen wird, doch in einem grundsätzlich anderen Medium gearbeitet wird. Enzyme unterliegen Regulationsprozessen, d.h. ihre katalytische Aktivität wird stark durch die Konzentration von Substraten, Produkten oder anderen gelösten Stoffen im Reaktionsmedium beeinflußt. Während das Reaktionsmedium in der Zelle sehr komplex aufgebaut ist, findet die erfindungsgemäße enzymatische Umsetzung in einem vergleichsweise einfachen, gegebenenfalls zelluntypischen Medium statt, das chemisch definiert sein kann.

Enzymreaktionen in der Zelle laufen unter idealen Bedingungen ab. Auf der anderen Seite kann man die erfindungsgemäße enzymatische Herstellung des Trisaccharids durchaus mit einem "Scale up" vergleichen, dessen allgemein bekannte Schwierigkeiten unter anderem auch darauf zurückzuführen sind, daß man nicht mehr unter den für den kleinen Maßstab idealen Bedingungen arbeiten kann. Trotz dieser vergleichsweisen Verschlechterung der Reaktionsbedingungen unter den erfindungsgemäßen Gegebenheiten, erhält man das Endprodukt in ausgezeichneten Ausbeuten.

Gegenüber der chemischen Synthese des Trisaccharids hat die enzymatische Synthese den großen Vorteil der geringeren Stufenzahl bei absoluter Stereospezifität und der sehr viel besseren Ausbeute.

Die Erfindung betrifft somit ein Verfahren zur Herstellung der Verbindung der Formel I,

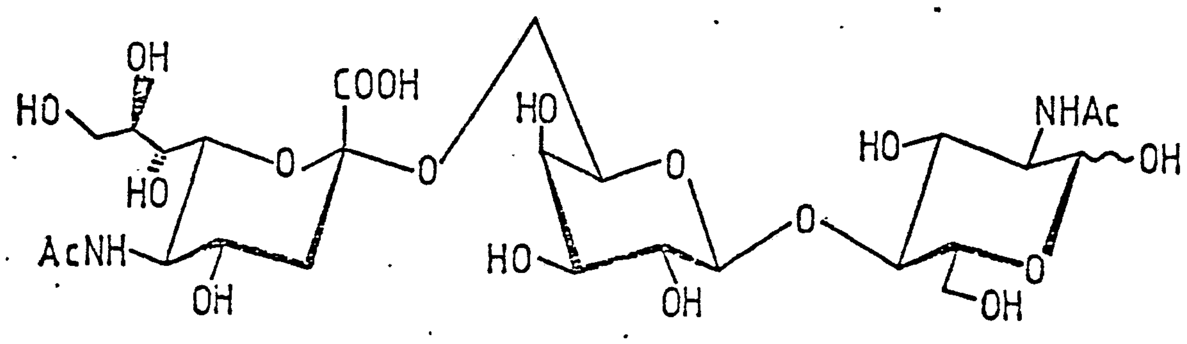

I

das dadurch gekennzeichnet ist, daß die Verbindung der Formel II

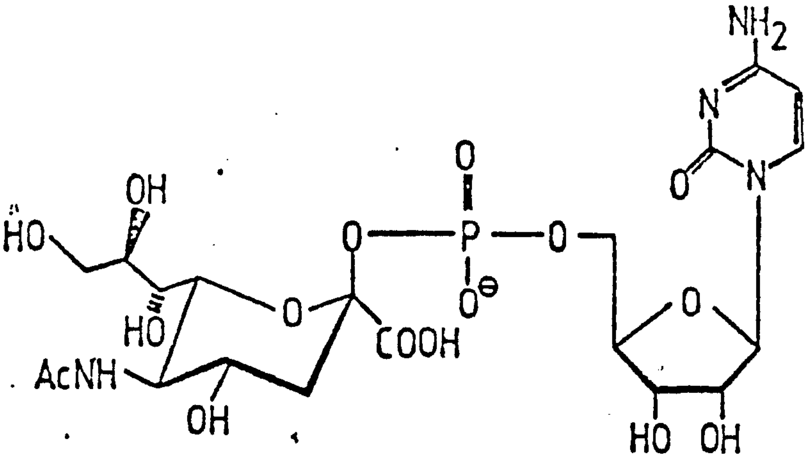

II

zusammen mit der Verbindung der Formel III

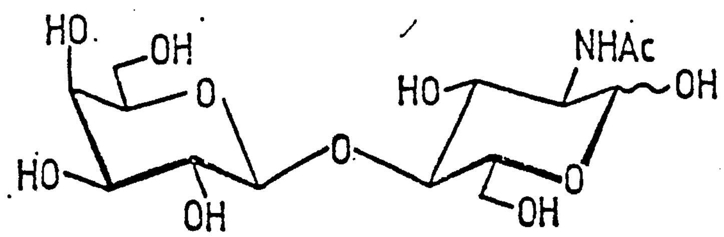

III

mit im wesentlichen reiner Sialyltransferase umgesetzt wird.

Im folgenden wird die Erfindung im einzelnen erläutert sowie in den Ansprüchen definiert.

Cytidin-5'-monophosphosialat (Verbindung der Formel II) kann beispielsweise nach einer Methode ähnlich der von Schauer präpariert werden. [R. Schauer, M. Wember u. C. Ferreira do Amaral, Hoppe Seyler's Z. Physiol. Chem. 353, 883 (1972)]. Das Enzym Cytidin-5'-monophosphosialat-Synthase [E.C. 2.7.7.43] wird aus dem Rohextrakt von Glandula submandibularis vom Rind grob gereinigt und frei oder immobilisiert eingesetzt. Wenn immobilisiert wird, ist Kieselgel als Träger, insbesondere aus Kostengründen, bevorzugt. Das Kieselgel wird dann z.B. zunächst durch Reaktion mit $\gamma$-Aminopropyltriethoxysilan und einem Überschuß Glutardialdehyd funktionalisiert. Das Enzym kann so durch Reaktion seiner Aminogruppen mit den Carbonylgruppen des Trägers fixiert werden.

Die Verbindung der Formel II entsteht z.B. durch Inkubation eines Ansatzes aus N-Acetylneuraminsäure und Cytidintriphosphat mit dem Enzym bei 20 bis 45°C und pH 8 bis 9,5. Es hat sich als vorteilhaft erwiesen, Cytidintriphosphat portionsweise zu dem Reaktionsansatz zu geben. Die Verbindung II wird nach beendeter Reaktion im allgemeinen zur weiteren Verwendung aus dem Reaktionsgemisch isoliert und gereinigt, beispielsweise durch Ionenaustausch oder Gelfiltration.

N-Acetyllactosamin (Verbindung der Formel III) kann nach der Methode von Whitesides [C.-H. Wong, S. Haynie u. G.M. Whitesides, J. Org. Chem. 47, 5416 (1982)] aus Glucose-6-phosphat, N-Acetylglucosamin, Uridindiphosphat und Phosphoenolpyruvat durch einen enzymatischen Synthesecyclus mit Kofaktorregenerierung hergestellt werden. Die für die Synthese erforderlichen sechs Enzyme können jedoch auch an dem kostengünstigeren Kieselgel fixiert werden. Die Verbindung III wird ebenfalls nach beendeter Reaktion im allgemeinen aus dem Reaktionsgemisch isoliert und dann zur Herstellung des Trisaccharids im wesentlichen rein eingesetzt.

Die Sialyltransferase [E.C. 2.4.99.1] kann mittels Affinitätschromatographie aus Colostrum (Biestmilch) isoliert werden [J.C. Paulson, J.E. Rearick u. R.L. Hill, J. Biol. Chem. 252, 2356 (1977)].

Zur Herstellung der Verbindung I werden die Verbindungen II und III in wäßriger Lösung mit im wesentlichen reiner Sialyltransferase im allgemeinen bei 20 - 45°C, insbesondere 35 - 37°C, und einem pH-Wert von 5 - 8, bevorzugt 6 - 7, inkubiert. Als wäßrige Lösung eignen sich grundsätzlich solche, die die Enzymaktivität nicht wesentlich beeinträchtigen. Bevorzugt werden Puffer, insbesondere Cacodylatpuffer eingesetzt.

Es hat sich als besonders günstig erwiesen, wenn die Verbindung II vorgelegt wird und das Enzym sowie die Verbindung I portionsweise in ca. 1 - 3 stündigem Abstand über einen Zeitraum von 10 - 30 Stunden, vorzugsweise ca. 20 Stunden, dem Reaktionsansatz zugegeben werden.

Die Aufarbeitung der Verbindung I kann anschließend durch Ionenaustauschchromatographie mit eventuell nachfolgender Gelfiltration erfolgen.

In den folgenden Beispielen wird die Erfindung weiter im Detail erläutert. Prozentangaben beziehen sich auf das Gewicht, wenn nicht anders angegeben.

## Beispiele

### 1. Herstellung der Verbindung II

30 g gefrorene Unterkieferdrüsen (Glandula submandibularis) vom Rind werden in dünne Scheiben zerschnitten und bei 4°C 1 Stunde mit 100 ml 0,1 M Tris Puffer und $10^{-4}$ M Mercaptoethanol geschüttelt. Dann wird zentrifugiert und durch Glaswolle filtriert. Der Rohextrakt wird auf eine mit Wasser äquilibrierte Diethylaminoethyl-Cellulose Säule (2,5x50 cm) aufgetragen, die vorher mit 2l 0,01 M Tris Puffer (pH 7,6) gewaschen wird. Es wird mit 2l 0,01 M Tris Puffer (pH 7,6), 0,075 M KCl eluiert. Die Fraktionen werden mit dem Test von Kean auf Enzymaktivität überprüft [E.L. Kean u. S. Roseman, Meth. Enzymol. 8, 208 (1966)]. Die Enzym-enthaltenden Fraktionen werden durch Gefriertrocknung konzentriert. 200 U (250 mg Protein) des auf diese Weise erhaltenen Enzyms werden analog der Methode von Wheetall [H.H. Wheetall, Meth. Enzymol. 44, 134 (1976)] an 5 g Kieselgel, Grace 332,250 Å, immobilisiert. Dazu werden zu 5 g Kieselgel 90 ml dest. Wasser und 10 ml γ-Aminopropyltriethoxysilan gegeben. Der pH-Wert wird mit 6N HCl auf pH 3 - 4 eingestellt. Danach werden die Reaktionspartner 2 Stunden in ein 75°C warmes Wasserbad gegeben. Dann wird über einen Büchnertrichter abfiltriert und mit 200 ml dest. Wasser gewaschen. Das Produkt wird 4 Stunden bei 115°C getrocknet. Nach dem Trocknen wird der Träger mit 200 ml einer 25 %igen Glutardialdehydlösung in 0,05 M $Na_2HPO_4$-Puffer (pH 7,0) versetzt und 2 Stunden gerührt. Danach wird abgesaugt und mit 500 ml dest. Wasser, 0,5 % NaCl-Lösung und 500 ml dest. Wasser gewaschen. Zu diesem reaktiven Träger werden die 250 mg des isolierten Enzyms in 10 ml $Na_2HPO_4$-Puffer (pH 7,0) gegeben und 2 - 4 Stunden inkubiert. Danach wird wieder mit Wasser, 0,5 % NaCl und Wasser gewaschen. Das fixierte Enzym kann bei 0 - 5°C aufbewahrt werden.

20 mg N-Acetylneuraminsäure werden mit 200 mg Cytidintriphosphat in 30 ml 0,1 M Tris Puffer, pH 9,0, 0,01 M $MgCl_2$ bei 37°C unter Wirkung des immobilisierten Enzyms zur Reaktion gebracht. Das Cytidintriphosphat wird portionsweise alle 30 Minuten zugesetzt. Nach 4 Stunden wird das fixierte Enzym abgesaugt und das Reaktionsgemisch auf einer Gelfiltrationssäule, BioGel/P 2 Agarose (70 x 2,5 cm), die vorher mit 1mM $NH_3$-Puffer (pH 8,5), äquilibriert wird, aufgetragen. Mit diesem Puffer wird auch eluiert, wobei 15 ml pro Glas gesammelt werden. Die Elutionsgeschwindigkeit beträgt 30 ml/h.

Das Eluat wird mit dem Assay nach Kean auf Produkt untersucht. Zu je 50 µl des Eluates werden 75 µl $NaBH_4$-Lösung (2,7 M in Wasser) gegeben. Es wird 15 Minuten bei Raumtemperatur geschüttelt. Dann werden 75 µl Aceton zugesetzt und abermals 15 Minuten geschüttelt. Nach Zugabe von 250 µl einer 0,2 M Natriumperiodatlösung in 9,0 M $H_3PO_4$ wird 20 Minuten bei Raumtemperatur stehengelassen. Schließlich wird noch pro Analyse je 1 ml einer 10 %igen Na-Arsenitlösung in 0,5 M $Na_2SO_4$ und 3 ml einer 0,6 % Thiobarbitursäurelösung in Wasser zugeführt. Dann wird 15 Minuten in einem kochendem Wasserbad erhitzt. Proben, die Cytidin-5'-monophosphosialat enthalten, verfärben sich violett. Die Extinktion kann bei 549 nm am Photometer gemessen werden. Die Fraktionen 8 - 12 enthalten Cytidin-5'-monophosphosialat. Nach der Gefriertrocknung beträgt die Ausbeute 29 mg (= 72%).

2. Herstellung der Verbindung III

Zu 50 ml eines 0,1 M $NaHCO_3$, 0,5 M NaCl Puffer (pH 8,0), werden 4 mmol Glucose-6-phosphat (1,43 g), 0,05 mmol Uridindiphosphat (27,7 mg), 4 mmol N-Acetylglucosamin (0,88 g), 4,2, mmol Phosphoenolpyruvat (0,87 g), 0,2 mmol $MnCl_2$ (25 mg) und 0,4 mmol $MgCl_2$ (40 mg) gegeben. Die Lösung wird desoxygeniert. Es werden folgende Mengen an Enzymen zugesetzt, die gemäß Beispiel 1 an Kieselgel fixiert werden:

Galactosyltransferase (EC 2.4.1.22) 2,5 U 7,0 g Träger
UDP-Galactosyl-Epimerase (EC 5.1.3.2) 10 U 3,0 g Träger
UDP-Glucose-Pyrophosphatase (EC 2.7.7.9) 10 U 1,0 g Träger
Phosphoglucomutase (EC 2.7.5.1) 50 U 1,0 g Träger
Anorganische Pyrophosphorylase (EC 3.6.1.1) 100 U 1,0 g Träger
Pyruvat Kinase (EC 2.7.1.40) 100 U 1,0 g Träger

Der pH-Wert beträgt 8,0 und wird mit einem automatischen Titrator konstant gehalten. Nach vier Tagen wird die Reaktion abgebrochen. Die überschüssigen, nicht umgesetzten Edukte werden mit 15 g Dowex 1X2 und Dowex 50 WX8 (vernetztes Polystyrol) entfernt. Das Reaktionsgemisch wird nach Konzentrierung über Bio-Gel/P 2 (2,7 x 70 cm), aufgetrennt. Die Elutionsgeschwindigkeit beträgt 0,6 ml/min; es werden 6 ml Fraktionen gesammelt. Das Eluat wird mittels Dünnschichtchromatographie überprüft. Laufmittel: n-Propanol/Essigsäure/Wasser (85:12:3; v:v:v)

$R_F$: Glucosamin 0,49
$R_F$: N-Acetyllactosamin 0,21
Die Ausbeute beträgt 405 mg (32 %).

3. Herstellung der Verbindung III

Die Sialyltransferase wird mittels Affinitätschromatographie aus Colostrum (Biestmilch) isoliert [J.C. Paulson, W.E. Rearick u. R.L. Hill, J. Biol. Chem. 252, 2356 (1977)]. Dazu werden 250 ml für 15 Minuten bei 4°C mit 30.000 g zentrifugiert. Die Fettschicht wird verworfen und das Colostrum wird anschließend über einen Zeitraum von 72 Stunden gegen 5 Volumen 10 mM Natriumcacodylatpuffer (pH 6,5), bei 4°C dialysiert. Danach erfolgt nochmals ein Zentrifugationsschritt. Dann wird über 25 ml einer Cytidindiphosphat-Agarosesäule gegeben, mit 300 ml 25 mM Na-Cacodylatpuffer gewaschen und - schließlich mit 25 ml einer 0,5 M NaCl-Lösung in 25 mM Cacodylatpuffer (pH 5,3) eluiert. Das Eluat wird aufgefangen und kann für die Reaktion direkt eingesetzt werden.

60 mg Cytidin-5'-monophosphosialat (Verbindung II, 0,2 mmol) und 32 mg N-Acetyllactosamin (Verbindung I, 0,1 mmol) werden in einem Puffer aus 10 ml 10 mM Na-Cacodylat, (pH 6,5), 2 mmol $MnCl_2$ und 10 mg Albumin mit 2,5 U Sialyltransferase [E.C. 2.4.99.1] 8 Stunden bei 37°C zur Reaktion gebracht.

Das Enzym und Cytidin-5'-monophosphosialat werden portionsweise alle 2 Stunden zugesetzt. Nach Beendigung der Reaktion wird das Reaktionsgemisch bei Raumtemperatur auf einer mit Phosphationen beladenen Dowex 1X2-Säule (2,5 x 70 cm) aufgetragen. Es wird mit 1 l Wasser nachgewaschen und dann mit 0,01 M $Na_3PO_4$ Puffer (pH 6,8) eluiert. Die Detektion des Eluats erfolgt mit Dünnschichtchromatographie auf HPTLC-Fertigplatten mit Lichrosorb-$NH_2$ Beschichtung.

Laufmittel: EtOH/1 M Ammoniumacetat (pH 7,4) (7/3; v/v)
$R_F$: N-Acetyllactosamin 0,66
Trisaccharid 0,43

Die gefriergetrocknete Trisaccharidfraktion wird auf einer Bio-Gel/P2 Säule (2,7 x 70 cm) entsalzt. Nach erneuter Gefriertrocknung erfolgt die Charakterisierung mittels $^1$H-NMR-Spektroskopie:

$^1$H-NMR (300 NHz, $D_2O$): $\delta$ = 5,22 (d, 1H, 1-H-GlcNAc, $\beta$-Form, $J_{1,2}$ = 1,2 Hz), 4,48 (d, 1H, 1-H-Gal, $J_{1,2}$ = 7,6 Hz), 1,71 (dd, 1H, 3a-H-Neu-5-Ac, $J_{3a,3e}$ = 13,3, $J_{3a,4}$ = 11,8 Hz), 2,68 (dd, 1H, 3e-H-Neu-5-Ac, $J_{3a,3e}$ = 13,3, $J_{3e,4}$ = 4,7 Hz), 2,03 (s, 3H, NAc von Neu-5-Ac), 2,05 (s, 3H, NAc von GlcNAc).

Ausbeute: 31 mg (52 %).

**Ansprüche**

1. Verfahren zur Herstellung der Verbindung der Formel I

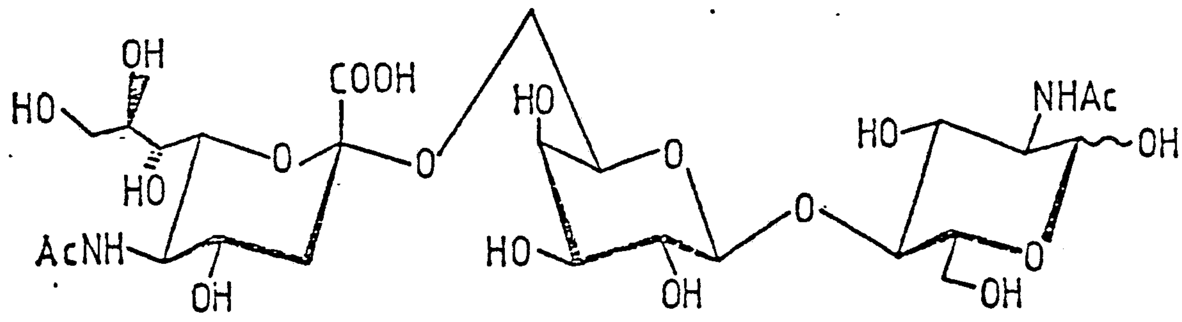

I

dadurch gekennzeichnet, daß die Verbindung der Formel II

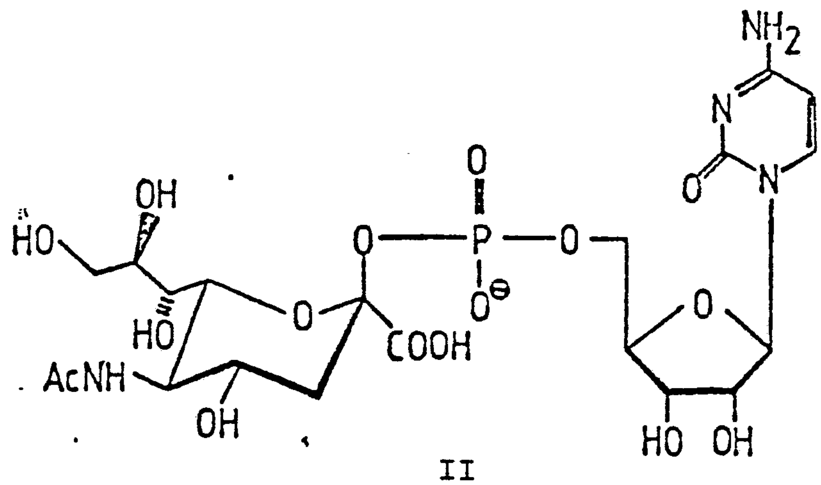

II

zusammen mit der Verbindung der Formel III

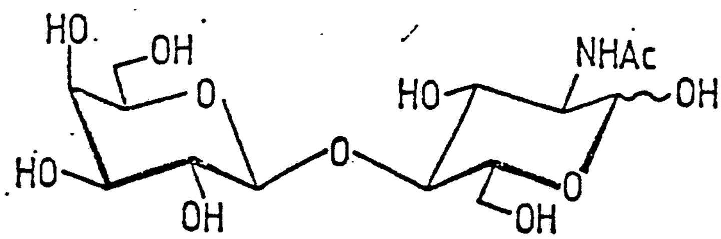

III

mit in wesentlichen reiner Sialyltransferase umgesetzt wird.

2. Verfahren nach Anspruch 1, dadurch gekennzeichnet, daß die Verbindungen der Formeln II und III im wesentlichen rein eingesetzt werden.

3. Verfahren nach Anspruch 1 oder 2, dadurch gekennzeichnet, daß die Reaktion in Puffer durchgeführt wird.

4. Verfahren nach einem oder mehreren der Ansprüche 1 bis 3, dadurch gekennzeichnet, daß die Reaktion bei 20 bis 45°C und einem pH-Wert von 5 bis 8 durchgeführt wird.

5. Verfahren nach Anspruch 4, dadurch gekennzeichnet, daß die Reaktion bei 35 bis 38°C durchgeführt wird.

6. Verfahren nach Anspruch 4, dadurch gekennzeichnet, daß die Reaktion bei einem pH-Wert von 6 bis 7 durchgeführt wird.

7. Verfahren nach einem oder mehreren der Ansprüche 1 bis 6, dadurch gekennzeichnet, daß das Enzym und die Verbindung II portionsweise zum Reaktionsansatz gegeben werden.

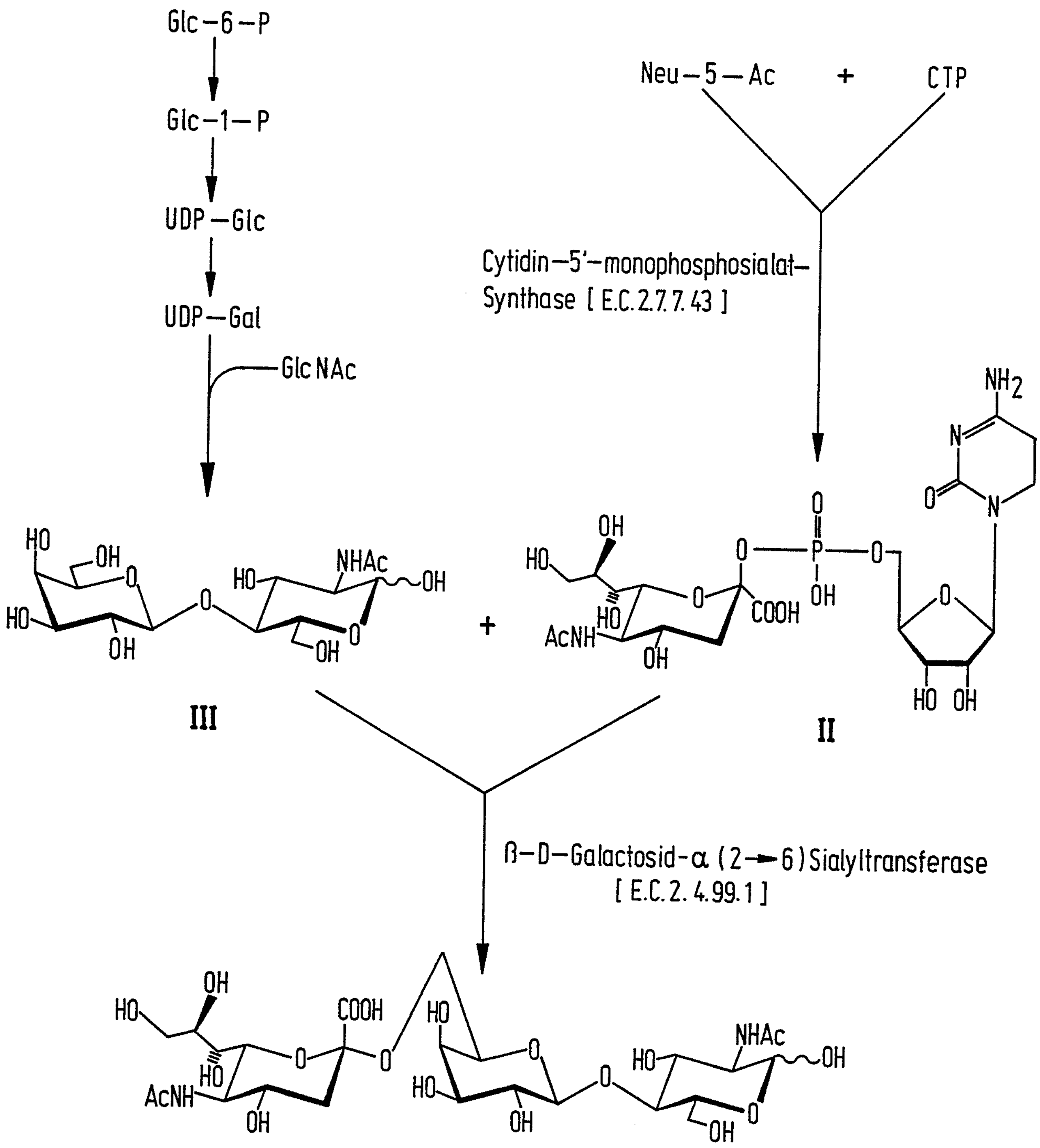
Glc–6–P
Glc–1–P
UDP–Glc
UDP–Gal
Glc NAc
Neu–5–Ac + CTP
Cytidin–5'–monophosphosialat–
Synthase [ E.C. 2.7.7.43 ]
ß–D–Galactosid–α (2→6) Sialyltransferase
[ E.C. 2. 4.99.1 ]

III

II

I